# EUROPÄISCHE PATENTSCHRIFT

(11) **EP 3 787 701 B1**
(45) Veröffentlichungstag und Bekanntmachung des Hinweises auf die Patenterteilung: **18.02.2026**
(21) Anmeldenummer: 19723342.2
(22) Anmeldetag: 03.05.2019
(51) Int. Cl.: A61L 27/16, A61L 27/50, A61B 17/17, A61K 6/00, A61L 24/06

(54) **HÄRTBARES RÖNTGENSICHTBARES MATERIAL**
CURABLE RADIOPAQUE SUBSTANCE
MATÉRIAU DURCISSABLE VISIBLE AUX RAYONS X

(30) Priorität: 04.05.2018 DE 102018206995
(43) Veröffentlichungstag der Anmeldung: 10.03.2021
(73) Patentinhaber: Merz Dental GmbH, 24321 Lütjenburg (DE)
(72) Erfinder: PFLESSER, Sebastian, 24143 Kiel (DE)
(74) Vertreter: Kunz, Herbert
(86) Internationale Anmeldenummer: PCT/EP2019/061330
(87) Internationale Veröffentlichungsnummer: WO 2019/211420

(56) Entgegenhaltungen:
- EP-A1- 1 366 774
- EP-A1- 1 430 913
- EP-A1- 2 153 812
- US-A- 5 024 232

## Beschreibung

Die vorliegende Erfindung betrifft ein härtbares und röntgensichtbares Material, einen daraus durch Polymerisation herstellbaren Werkstoff, Verfahren zur Herstellung des härtbaren Materials und des gehärteten Werkstoffs und die Verwendung des härtbaren Materials bzw. des gehärteten Werkstoffs. Das härtbare Material bzw. der gehärtete Werkstoff kann u.a. in der Orthopädie als sog. Knochenzement insbesondere aber als Zahnfüllungsmaterial, Dentalzement, dentales Unterfüllungsmaterial, fließfähiges Kompositmaterial (Flow-Material), Kronen- und Brückenmaterial, zur Herstellung von Inlays, Onlays und Stumpfaufbaumaterialien sowie in der Röntgendiagnostik als Bohrschablone eingesetzt werden. Daneben betrifft die vorliegende Erfindung die Verwendung des härtbaren Materials ganz allgemein als Konstruktionsmaterial in einem generativen, ein digitales Datenmodell verwendenden Fertigungsverfahren.

Für härtbare - röntgensichtbare - Materialien wurden während der letzten Jahrzehnte in der modernen Medizin - wie z.B. auf dem Gebiet der Orthopädie oder in der Zahnmedizin - weite Anwendungsfelder erschlossen. Als repräsentative Beispiele seien an dieser Stelle lediglich Knochenzemente und Füllungsmaterialien bzw. Restaurationsmaterialien genannt. Derartige Materialien basieren auf organischen Polymeren, wobei die Knochenzementmischung polymerisierbare Monomere sowie einen Initiator und/oder Aktivator zum Auslösen der Polymerisation enthält, so dass die Verfestigung auf dem Wege der - kalten - Polymerisation erfolgt.

So basiert ein verbreitet angewandter organischer Knochenzement beispielsweise auf Polymethylmethacrylat (PMMA), der durch Polymerisation des Monomers Methylmethacrylat (MMA) erhalten wird. Kommerzielle PMMA-Knochenzemente werden als zu mischende Zweikomponentensysteme angeboten.

In der orthopädischen wie auch insbesondere in der zahnärztlichen Praxis ist es oft von entscheidender Bedeutung, nicht-natürliches (d.h. künstliches) Material eines zuvor behandelten Knochens oder Zahns vom verbliebenen natürlichen Knochen- bzw. Zahnmaterial eindeutig unterscheiden zu können. Eine solche Unterscheidung wird zum Beispiel durch eine röntgenographische Untersuchung ermöglicht. Anhand einer Röntgenaufnahme können beispielweise in der Füllungstherapie Randspalten erkannt werden. Der Zahnarzt wird insbesondere in die Lage versetzt, bereits kleine Randspalten zwischen einem Füllungskomposit (als Beispiel eines nicht-natürlichen bzw. künstlichen Dentalmaterials) und dem umgebenden natürlichen Zahnmaterial zu identifizieren und - falls notwendig - genauestens zu excavieren. Hierzu ist es jedoch notwendig, dass das Füllungskomposit (und ganz generell künstliche Materialien, die in der Osteosynthese Verwendung finden neben Dentalmaterialien) eine ausreichend hohe Röntgenopazität besitzen, um während der Röntgenaufnahme Röntgenstrahlen ausreichend stark absorbieren zu können. Diese Absorption sorgt in einem Röntgenbild für den notwendigen Kontrast, der letztendlich eine Unterscheidung zwischen dem natürlichem Zahnmaterial einerseits und Füllungskomposit (bzw. dem künstlichem (Dental)material) andererseits unterscheiden zu können. Das Füllungskomposit (bzw. ganz allgemein Knochen- bzw. Zahnbestandteile aus künstlichem (Dental)material) ist in einem Röntgenbild regelmäßig anhand eines geringeren Schwärzegrades erkennbar. Eine hinreichende Röntgenopazität des künstlichen Dentalmaterials erlaubt somit sehr häufig eine sichere Unterscheidung zwischen künstlichem (Dental)material und natürlichem Knochen- bzw. Zahnmaterial.

Die natürliche Röntgenopazität eines menschlichen Zahns betragt üblicherweise 2 mm Aluminium (Al) oder weniger (Dentin ca. 1,5 mm Al, Zahnschmelz ca. 2 mm Al). Daher sollte ein röntgenopakes, künstliches Dentalmaterial im Allgemeinen mindestens einen Wert größer 2,5 mm Al besitzen. Beispielsweise bedeutet ein Wert von 10 mm Al, dass ein 1 mm dicker Probekörper aus röntgenopakem, künstlichem, gehärtetem Dentalmaterial zu einer Schwärzung auf einem Röntgenfilm führt, die identisch ist mit der Schwärzung, die durch einen Probekörper aus Aluminium hervorgerufen wird, der eine Dicke von 10 mm besitzt.

Besonders bevorzugte Röntgenopazitäten liegen in einem Bereich zwischen 3,0 und 5,0 mm Al. Ein künstliches Dental(material) (z.B. ein Füllungskomposit) kann in einem Röntgenbild umso besser vom natürlichen Zahnmaterial unterschieden werden, je höher die Röntgenopazität des künstlichen Dentalmaterials ist. Ein bekanntes, aber auch umstrittenes geeignetes künstliches Dentalmaterial ist Amalgam, dessen Röntgenopazität bis über 10 mm Aluminium (Al) betragen kann.

Auf dem Gebiet der Röntgendiagnostik wurden in den letzten Jahren erhebliche Fortschritte erzielt, die eine immer exaktere Unterscheidung zwischen künstlichem osteosynthetischen Material bzw. Dentalmaterial und umgebenden natürlichen Materialien erlauben.

In den letzten Jahren hat sich insbesondere ein Verfahren in den zahnärztlichen Praxen etabliert, das als Digitale Volumentomographie (DVT) bekannt ist. Ein DVT-Gerät besteht im Wesentlichen aus einer Röntgenquelle und einem gegenüberliegenden Detektor (z.B. Flatpanel-Detektor). Bei der DVT wird ein Untersuchungsobj ekt von einem Konus-förmigen oder pyramidalen, meist gepulsten Röntgenstrahl (Röntgenblitz) durchdrungen. Daher hat sich im englischen Sprachraum die Bezeichnung CBCT (cone beam computer tomography) durchgesetzt. Auf der der Röntgenquelle gegenüberliegenden Seite werden die durch das Untersuchungsobjekt abgeschwächten Signale als zweidimensionale Projektion auf dem Detektor detektiert. Während der Untersuchung rotiert die Einheit aus Röntgenquelle und Detektor (Gantry) um das Untersuchungsobjekt, um eine Vielzahl von Einzelaufnahmen zu erstellen. In jeder Einzelaufnahme werden abgeschwächte Grauwerte-Röntgenbilder als 2D-Projektion gewonnen. Aus diesen Einzelaufnahmen wird mittels Rückprojektion eine dreidimensionale Rekonstruktion (Grauwert-Koordinatenbild, Volumengrafik) errechnet, die die anatomischen Strukturen des Untersuchungsobjektes in Form von Voxeln unterschiedlicher Graustufen abbildet. Im Ergebnis kann die dreidimensionale Rekonstruktion entweder in Form einzelner, zweidimensionaler Schnittbilder (Tomogramme) oder in einer 3D-Ansicht betrachtet werden.

Als besonders vorteilhaft haben sich DVT-generierte Bilder in der dentalen Implantologie erwiesen. Sie erlauben eine besonders sorgfältige Planung von Implantaten und deren Einsetzung unter Berücksichtigung des zur Verfügung stehenden Knochenangebots. Auch bei der Lokalisation von Weisheitszähnen in Vorbereitung chirurgischer Maßnahmen haben sich DVT-generierte Bilder als sehr zuverlässig erwiesen.

Aus dem Stand der Technik ist beispielsweise der Einsatz Barium-haltiger Gläser oder schwerlöslicher Ytterbiumfluorid-Partikel zur Röntgenkontrastierung hochviskoser Kompositmaterialien - wie sie als Zahnfüllungs- /Restaurationsmaterial eingesetzt werden - bekannt.

So offenbart die deutsche Offenlegungsschrift DE 24 46 547 A1 die Verwendung eines Bariumsilicatglases als "Mikrofüller" zur Erlangung einer Röntgenopazität von Kunstharzmassen. die europäische Patentschrift EP 0 717 976 B1 lehrt die Verwendung eines Bariumaluminium-Borosilicatglas-Mikrofüllers mit einer Teilchengröße im Bereich von 0,7 µm.

Induziert durch den Umstand, dass sich die Bariumionen, die aus den Gläsern freigesetzt werden, als toxisch erwiesen, wurden in der Folge bariumfreie Gläser entwickelt, in denen u.a. Strontium-Verbindungen als Kontrastmittel verwendet werden.

Daneben offenbart die deutsche Patentschrift DE 43 23 143 C1 die Verwendung von Strontiumsilicaten als Mikrofüller (microfiller). Des Weiteren lehrt die europäischen Offenlegungsschrift EP 0 511 868 A2 den Einsatz von Strontiumphosphat/Strontiumapatit-Systemen mit einer Teilchengröße von 10 µm.

Daneben wurden für gezielt bariumfreie röntgensichtbare Dentalgläser Oxidgemische sowie Mischoxide z.B. der Elemente Lanthan, Wolfram und Zirkonium im Stand der Technik - wie z.B. in der internationalen Patentanmeldung WO 2007/048670 A2 - vorgeschlagen.

Des Weiteren wurden Strontium/Zink/Zirkonium-Silicate als Füller bzw. Füllstoffe - z.B. gemäß der Lehre der deutschen Patentschrift DE 19 849 388 C2 - eingesetzt.

Zusätzlich wird im Stand der Technik auch die Verwendung von reinen Mischoxiden in hochviskosen Systemen offenbart - wie z.B. gemäß der Lehre der Deutschen Offenlegungsschrift DE 34 21 155 A1, welche die Verwendung Oxiden eines Strontium/Lanthan/Wolfram-Kombinationsmikrofüllstoffs offenbart. Bei einer derartigen Verwendung werden die gehärteten Komposite jedoch oftmals opak und verlieren in der Regel an Risszähigkeit sowie vorteilhafte Eigenschaften anderer mechanischer Charakteristika. Eine Verkleinerung der Füllkörper in den oberen Nanometerbereich hinein verschafft hier nur eine Verbesserung hinsichtlich der optischen Eigenschaften sowie bei der Politur.

Alternativ zu den Gläsern werden gemäß der europäischen Offenlegungsschrift EP 0 238 025 A2 ebenfalls schwerlösliche komplexe Fluoridverbindungen vorgeschlagen - wie z.B. BaZrF₆ und SrZrF₆ sowie YF₃ - neben schwerlöslichen Seltenerdfluoriden, wie sie z.B. in der europäischen Offenlegungsschrift EP 0 189 540 A2 offenbart werden.

Des Weiteren offenbart die internationale Patentanmeldung WO 2002/055028 A2 die Verwendung von Lanthanidoxiden als röntgensichtbare Mikrofüller, während die deutsche Offenlegungsschrift DE 24 58 380 A1 La-, Hf-, Sr- und Ta-Oxide und deren Carbonate in Gläsern verwendet, um eine befriedigende Röntgensichtbarkeit zu erzielen zu können. Gemäß der Lehre der deutschen Offenlegungsschrift DE 29 35 810 A1 werden Thoriumund/oder Tantaloxid in Zahnfüllungsmaterialien vorgeschlagen. Dieser Lösungsansatz konnte sich allerdings - auf Grund der Radioaktivität des Thoriums neben seiner Schwermetall-Toxizität - in der praktischen Anwendung nicht etablieren.

Des Weiteren offenbaren die europäische Offenlegungsschrift EP 0 143 362 A2 sowie die deutsche Offenlegungsschrift DE 44 19 386 A1 die Verwendung von reaktiven (Acryl-)Monomeren, die kovalent gebundenes Brom oder Jod, die eine gewisse Röntgensichtbarkeit generieren können, enthalten.

Gemäß des jüngeren Standes der Technik kommen verstärkt Nanopartikel in den in Rede stehenden Komposit-Systemen, zum Einsatz, da diese bei immer kleiner werdenden Teilchengrößen, die optischen Eigenschaften immer weniger negativ beeinflussen. So offenbart die europäische Patentanmeldung EP 1 711 433 die Synthese sowie die Verwendung von nanopartikularen Sprühflammenmischoxiden auf der Basis von Siliciumdioxid (SiO₂) und Seltenerdoxiden in Dentalkompositen, während gemäß der Offenbarung der deutschen Offenlegungsschrift DE 10 2006 045 628 A1 diese Sprühflammenmischoxide bereits in einem röntgenopaken Dentaladhäsiv zur Anwendung gebracht wurden.

Die deutsche Offenlegungsschrift DE 10 2015 220 373 A1 offenbart daneben die Verwendung von Partikeln aus Bariumsulfat und Ytterbiumfluorid mit einer Teilchengröße von 25 - 120 nm in der Form eines röntgenopaken Füllstoffs in härtbaren Dentalmaterialien und lehrt des Weiteren die Verwendung eines solchen Materials für den Bereich der generativen Fertigung, vorzugsweise unter Anwendung des 3D-Drucks.

EP 1 366 774 A offenbart eine Knochenzementmischung, gebildet aus einer ein Röntgenkontrastmittel enthaltenden Polymerkomponente und einer Monomerkomponente, wobei das Röntgenkontrastmittel als Polymer mit daran chemisch gebundenen Verbindungen röntgenopaker Elemente (z.B. Zirkonyl-di-methacrylat) ausgebildet ist.

Die oben diskutierten, im Stand der Technik beschriebenen Systeme füllstoffhaltigen Charakters bergen jedoch das Risiko in sich, dass sie bei der Übertragung auf niedrig-viskose Harze - wie sie bei der additiven Fertigung im STL- oder DLP-Verfahren eingesetzt werden - dazu führen, dass sie sich auf Grund der Gravitationskraft im Laufe der Zeit absetzen und/oder sich ein Konzentrationsgradient einstellt, der in der Folge im Laufe der Zeit dazu führt, dass das Endprodukt keine reproduzierbaren Eigenschaften insbesondere in Bezug auf die Röntgenopazität aufweist.

Ähnlich, wie in der klassischen Röntgendiagnostik, spielt aber auch in der modernen Orthopädie sowie in der Zahnmedizin die Röntgenopazität eine entscheidende Rolle für die Unterscheidung zwischen künstlichem Dental- bzw. Knochenmaterial und natürlichem Zahn- bzw. Knochenmaterial. Es besteht somit ein ständiges Bedürfnis, die Röntgenopazität härtbarer Dental- bzw. Knochenmaterialien, die mittels Polymerisation geeigneter polymerisierbarer Monomere zu den entsprechenden gehärteten, künstlichen Dental- bzw. Knochenmaterialien weiterverarbeitet werden, vorteilhaft einstellen zu können.

Die Aufgabe, die der vorliegenden Erfindung zu Grunde liegt, wird erfindungsgemäß durch die in Anspruch 1 beanspruchte Zusammensetzung ein für härtbares und röntgensichtbares Material gelöst. Daneben betrifft die vorliegende Erfindung ein aus dem härtbaren Material durch Polymerisation herstellbares gehärtetes Material, Verfahren zur Herstellung des härtbaren sowie des gehärteten Materials und die Verwendung des härtbaren sowie des gehärteten Materials, d.h. des Werkstoffs.

Das härtbare Material bzw. der Werkstoff kann u.a. in der Orthopädie als sog. Knochenzement insbesondere aber auch in der Zahnmedizin als Zahnfüllungsmaterial, Dentalzement, dentales Unterfüllungsmaterial, fließfähiges Kompositmaterial (Flow-Material), Kronen- und Brückenmaterial, zur Herstellung von Inlays, Onlays und als Stumpfaufbaumaterial sowie in der Röntgendiagnostik als Bohrschablone eingesetzt werden.

Daneben wurde gefunden, dass die erfindungsgemäßen Materialien bzw. Werkstoffe auch durch Eigenschaften hervorstechen, die sie für einen Einsatz auf dem Gebiet der optischen Geräte hochinteressant erscheinen lassen.

In einer besonderen Anwendungsform kann das härtbare Material als Baumaterial in einem generativen, ein digitales Datenmodell verwendenden Fertigungsverfahren, vorzugsweise im 3D-Druck und insbesondere zur Herstellung eines Erzeugnisses für die Verwendung im Bereich der Optik, vorzugsweise zur Herstellung von Linsen und/oder Filtern eingesetzt werden

Das erfindungsgemäße härtbare Material für die Herstellung eines Werkstoffes ist herstellbar durch das Mischen von folgenden Ausgangsmaterialien:
- ein oder mehrere polymerisierbare(s) Monomer(e), eine oder mehrere Strontium-, Zirkonium-, Blei-, Barium-, Bismut- oder Seltenerdverbindung(en), die in dem Monomeren oder in dem Monomerengemisch löslich ist/sind,
- ein oder mehrere Härtungsinitiator(en)
- ein oder mehrere Hilfsstoffe, dadurch gekennzeichnet, dass der Hilfsstoff eine aromatische Carbonsäure oder eine Aralkylcarbonsäure ist, womit die Aralkylcarbonsäure eine substituierte oder unsubstituierte, gesättigte oder ungesättigte Carbonsäure ist, womit vorzugsweise die Carbonsäure Phenylessigsäure oder 3-Phenylpropionsäure oder trans-Zimtsäure ist.

Bevorzugt wird wenigstens eines der polymerisierbaren Monomere aus der Gruppe der radikalisch härtbaren Monomere ausgewählt, worunter besonders bevorzugt wenigstens eines der polymerisierbaren Monomere aus der Gruppe der Acrylsäure, der Acrylate, der Methacrylsäure oder der Methacrylate bzw. aus deren Derivaten ausgewählt ist.

Beispiele für geeignete radikalisch härtbare Monomere der Acrylsäure bzw. der Methcrylsäure sind: Methylmethacrylat, Ethylmethacrylat, Isopropylmethacrylat, 2-Hydroxyethylmethacrylat, 3-Hydroxypropylmethacrylat, 2-Hydroxy-1,3-dimethacryloxypropan, n-Butylmethacrylat, Isobutylmethacrylat, Hydroxypropylmethacrylat, Glycidylmethacrylat, 2-Methoxyethylmethacrylat, 2-Ethylhexylmethacrylat, Benzylmethacrylat, 2,2-Bis-(methacryloxyphenyl)propan, 2,2-Bis-[4-(2-hydroxy-3-methacryloxypropoxy)phenyl]propan, 2,2-Bis-(4-methacryloxydiethoxy-phenyl)propan, 2,2-Bis-(4-methacryloxypolyethoxyphenyl)propan, Ethylenglykoldimethacrylat, Diethylenglykol-dimethacrylat, Triethylenglykoldimethacrylat, Butylenglykoldimethacrylat, Neopentylglykol-dimethacrylat, 1,3-Butandioldimethacrylat, 1,4-Butandioldimethacrylat, 1,6-Hexandioldimethacrylat, Trimethylolpropantrimethacrylat, Trimethylolethantrimethacrylat, Pentaerythrittrimethacrylat, Trimethylolmethantrimethacrylat, Pentaerythrittetramethacrylat sowie Methacrylate aufweisend eine Urethanbindung in ihrer derivatisierten Verbindung.

Ganz besonders bevorzugt werden folgende härtbare Materialien bzw. Dentalmaterialien eingesetzt, die ausgewählt sind aus der Gruppe umfassend Methacrylsäure, Butyldiglycolmethacrylat, Urethandimethacrylat, iso-Bomylmethacrylat, Tetrahydrofurfurylmethacrylat, 1,4-Butandiol-dimethacrylat, 2-[[(Butylamino)carbonyl]oxy]ethylacrylat, Bisphenol-A-dimethacrylat und/oder Methylmethacrylat.

Die genannten Monomere werden als Hauptbestandteil z.B. von Dentalmaterialien zur Polymerisation mit wenigstens einem Härtunginitiator für die radikalische Polymerisation und ggf. auch mit zusätzlichen Monomeren, und mit einer oder mehreren weiter unten beschriebenen Strontium-, Zirkonium-, Blei-, Barium-, Bismut- oder Seltenerdverbindung(en) und ggf. mit Hilfsstoffen gemischt. Die so erhaltenen Gemische können auf dem Wege der radikalischen Polymerisation gehärtet werden.

Sowohl die härtbaren Zusammensetzungen als auch die gehärteten Produkte - Werkstoffe - sind Gegenstand der vorliegenden Erfindung.

Zu den Metallen der "Seltenen Erden" des PSE gehören die chemischen Elemente der 3. Nebengruppe des Periodensystems - mit Ausnahme des Actiniums - und die Lanthanoide - insgesamt also 17 Elemente. Nach den Definitionen der anorganischen Nomenklatur heißt diese Gruppe chemisch ähnlicher Elemente auch Seltenerdmetalle. Die davon abgeleiteten Verbindungen sind u.a. Gegenstand der vorliegenden Erfindung gemäß Anspruch 1. Die erfindungsgemäß eingesetzten Seltenerdverbindungen leiten sich einmal von den leichteren Seltenerdmetallen - wie Scandium (Sc, 21), Lanthan (La, 57), Cer (Ce, 58), Praseodym (Pr, 59), Neodym (Nd, 60), Promethium (Pm, 61), Samarium (Sm, 62) und Europium (Eu, 63) ab - und zum anderen von den sog. schwereren Seltenerdmetallen - wie Yttrium (Y, 39), Gadolinium (Gd, 64), Terbium (Tb, 65), Dysprosium (Dy, 66), Holmium (Ho, 67), Erbium (Er, 68), Thulium (Tm, 69), Ytterbium (Yb, 70), Lutetium (Lu, 71).

Daneben umfasst die vorliegende Erfindung in dem Monomeren bzw. in dem Monomerengemsich lösliche Verbindungen der Elemente Strontium (Sr, 38), Zirkonium (Zr, 40) Blei (Pb, 82) Barium (Ba, 56) und Wismut bzw. Bismut (Bi, 83).

Als Initiatoren für die radikalische Polymerisation eignen sich die aus dem Stand der Technik wohlbekannten Initiatoren für die Heiß-, Kalt- und Photohärtung. Geeignete Initiatoren werden beispielsweise in der Encyclopedia of Polymer Science and Engineering, Vol. 13, Wiley-Interscience Publishers., New York 1988 genannt.

Gängige thermische Initiatoren sind zum Beispiel Azoverbindungen, wie Azobis(isobutyronitril) (AIBN) oder Azobis(4-cyanovaleriansäure) oder Peroxide, wie Dibenzoylperoxid, Dilaurylperoxid, tert.-Butylperoctoat, tert.-Butylperbenzoat oder Di(tert.-butyl)peroxid.

In Sinne der vorliegenden Erfindung bevorzugte UV-Härtungsinitiatoren werden durch Verbindungen aus der Gruppe der Phosphinoxide, bevorzugt Diphenyl-(2,4,6-trimethylbenzoyl)phosphinoxid (TPO) und/oder 2,4,6-Trimethylbenzoylphenylphosphinat (TPO-L) und/oder Bis(2,4,6-trimethylbenzoyl)-phenylphosphinoxid (BAPO) und/oder Campherchinon und/oder eine Verbindung aus der Gruppe der Thioxanthone verkörpert.

Unter Hilfsstoffen werden im Sinne der vorliegenden Erfindung organische Säuren verstanden, wobei die organische Säure durch eine aromatische Carbonsäure oder durch eine Aralkylcarbonsäure verkörpert wird.

Dabei kann die Aralkylcarbonsäure durch eine verzweigte oder unverzweigte Carbonsäure oder durch eine substituierte oder unsubstituierte, gesättigte oder ungesättigte Carbonsäure verkörpert werden.

Ganz besonders bevorzugt werden Aralkylcarbonsäuren ausgewählt aus der Gruppe umfassend Phenylessigsäure, 3-Phenylpropionsäure und trans-Zimtsäure.

Praktischerweise werden die Strontium-, Zirkonium-, Blei-, Barium-, Wismut- und/oder Seltenerdverbindung(en) in Form von Verbindungen bereitgestellt, aus denen *in-situ* die entsprechenden in den Monomeren löslichen Verbindungen in Konzentrationen in dem späteren Polymer entstehen, die eine Röntgensichtbarkeit ermöglichen.

In einer bevorzugten Ausführungsform werden die Blei-, Barium-, Wismut- und/oder Seltenerdverbindung(en) bereits in Form ihrer Verbindungen bzw. Komplexe bereitgestellt, die in den Monomeren bzw. Monomerengemisch(en) löslich sind. - Durch das Vorliegen von Metallionen in Lösung entfällt das Problem einer Sedimentation bei der für die generative Fertigung erforderlichen niedrigen Viskosität, wie es z.B. bei dem Einsatz von Füllstoffen auftritt. In der Konsequenz können die Lösungen bzw. Druckmaterialien problemlos gelagert werden mit der Folge, dass bei den auf Ink-Jet basierten Systemen die Gefahr einer Verstopfung der Druckerdüsen durch agglomerierte Partikel vermieden werden kann.

Des Weiteren enthalten die Materialien keine Partikel, welche die Photopolymerisation durch Absorption und Streuung des eintreffenden Lichtes stören können.

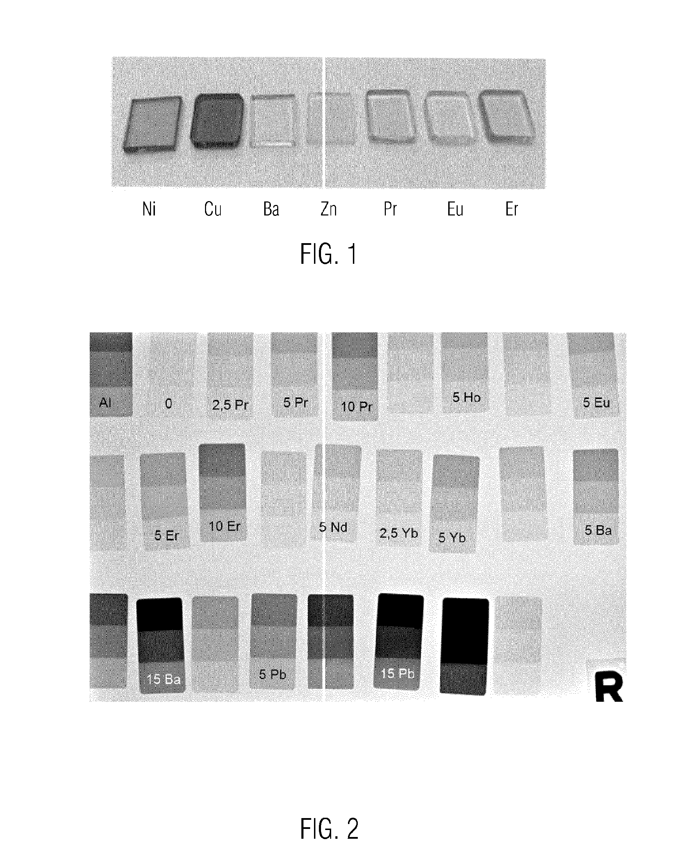

Die erhaltenen, polymerisierten bzw. gehärteten Werkstoffe sind glasklar und weisen keine Partikel auf, die als Fehlstellen betrachtet werden können und die somit die mechanischen Eigenschaften verschlechtern könnten, wie Fig. 1 für verschiedene Elemente belegt (Nickel und Kupfer gehören nicht zur vorliegenden Erfindung).

Die nach der Polymerisation erhaltenen makromolekularen Werkstoffe lassen sich daneben hervorragend polieren, da sie keine störenden Füllstoff-Partikel enthalten.

Die im Polymer gelösten Röntgenkontrastmittel liegen immer homogen verteilt in der Harzmischung und den polymerisierten Materialien vor, wodurch eine hervorragende Reproduzierbarkeit gewährleistet wird, was durch Fig. 2 belegt wird.

In Fig. 2 werden in der ersten Reihe Röntgenaufnahmen (durchgeführt mit einer an der Röntgenröhre anliegenden Spannung von 55 kV) von beispielhaft ausgewählten Proben (entsprechend gekennzeichnet) gezeigt. Die erste Reihe der Aufnahme der Probenkörper beginnt mit dem Standard Aluminium (Al) gefolgt von einer Probe, die gar keinen Metallgehalt aufweist (0). Es folgen Aufnahmen von Proben mit einem Gehalt von 5 und 10 Gew.-% Praseodym (entsprechend mit 5 Pr und 10 Pr gekennzeichnet).

In der zweiten Reihe werden Röntgenaufnahmen von Probekörpern enthaltend 5 und 10 Gew.-% Erbium (5 Er 5 und 10 Er) wiedergegeben, gefolgt von Aufnahmen von Proben enthaltend jeweils 5 Gew.-% Ytterbium (5 Yb) und Barium (5 Ba).

Die letzte Reihe zeigt u.a. die Röntgenaufnahme einer Probe, die 5 Gew.-% Blei (5 Pb) enthält

Es können somit auch komplexe röntgensichtbare Konstruktionen erstellt werden, die in klassischer Handarbeit, z.B. mit Autopolymerisaten darstellbarbar sind.

Die generativ hergestellten Konstruktionen passen wie vorgesehen, da der auftretende Polymerisationsschrumpf durch die CAD/CAM-Software im Voraus berücksichtigt bzw. mit einberechnet wird.

Durch die Verwendung polymerisierbarer Strontium-, Zirkonium-, Barium-, Blei-, Wismutoder Seltenerdverbindungen wird die Migration signifikant herabgesetzt.

Letztendlich verfügen die erfindungsmäßen Systeme bzw. härtbaren Materialien verfügen über eine einstellbare, niedrige Viskosität im Bereich von 500 - 3.000 mPas, während die aus dem Stand der Technik bekannten Komposite deutlich oberhalb von 40.000 mPas angesiedelt sind.

### Beispiele:

1. Herstellung von röntgensichtbarem, Praseodym-haltigen Polymerisat für die Herstellung von Borschablonen auf einem DLP-Drucker mit 385 nm. (nicht erfindungsgemäß)
1,21 g Praseodymcarbonat
5,03 g Methacrylsäure
2,03 g Butyldiglycolmethacrylat
6,96 g Urethandimethacrylat
0,31 g TPO-L
Die Mischung ergibt eine klare, grüne Lösung, die nach der Polymerisationsreaktion ein glasklares, grünes Plättchen liefert. Die Röntgensichtbarkeit beträgt etwa 70 % A1 bei etwas weniger als 5 % Pr.
2. Herstellung von röntgensichtbarem, Europium-haltigen Polymerisat

| | |
|---|---|
| 1,22 g | Europiumcarbonat |
| 4,06 g | 3-Phenylpropionsäure |
| 2,18 g | Methacrylsäure |
| 2,60 g | *iso*-Bornylmethacrylat |
| 5,32 g | Urethandimethacrylat |
| 0,37 g | TPO-L |

Die Mischung ergibt eine klare, leicht gelbe Lösung, die nach der Polymerisationsreaktion ein glasklares, gelbliches Plättchen liefert. Die Röntgensichtbarkeit beträgt etwa 73 % A1 bei etwas weniger als 5 % Eu.
3. Herstellung von röntgensichtbarem, Erbium-haltigem Polymerisat

| | |
|---|---|
| 2,34 g | Erbiumcarbonat |
| 2,51 g | 3-Phenylpropionsäure |
| 2,91 g | Phenylessigsäure |
| 3,01 g | Methacrylsäure |
| 2,00 g | *iso*-Bornylmethacrylat |
| 3,17 g | Butylglycolmethacrylat |
| 0,27 g | TPO |

Die Mischung ergibt eine klare, rosafarbene Lösung, die nach der Polymerisationsreaktion ein glasklares, rosa Plättchen liefert. Die Röntgensichtbarkeit beträgt etwa 85 % A1 bei 10 % Er.
4. Herstellung von röntgenopakem Blei-haltigem Polymerisat zur Verwendung als Druckmaterial, z.B. für die Herstellung röntgenabsorbierender Fenster.

| | |
|---|---|
| 3,28 g | Bleioxid |
| 3,02 g | 3-Phenylpropionsäure |
| 3,24 g | Methacrylsäure |
| 0,66 g | *iso*-Bornylmethacrylat |
| 4,93 g | Urethandimethacrylat |
| 0,33 g | TPO-L |

Die Mischung ergibt eine klare, leicht bräunliche Lösung, die nach der Polymerisationsreaktion ein glasklares, leicht braun gefärbtes Plättchen liefert. Die Röntgensichtbarkeit beträgt 162 % A1 bei etwas weniger als 20 % Blei.
5. Herstellung von röntgensichtbarem, Barium-haltigen Polymerisat I

| | |
|---|---|
| 1,26 g | Bariumhydroxid, wasserfrei |
| 2,99 g | Methacrylsäure |
| 0,99 g | *trans-Zimtsäure* |
| 4,03 g | Butyldiglycolmethacrylat |
| 2,08 g | Methacrylsäureanhydrid |
| 7,78 g | Urethandimethacrylat |
| 0,36 g | TPO |

Die Mischung ergibt eine klare, leicht gelbliche Lösung, die nach der Polymerisationsreaktion ein glasklares, leicht gelblich gefärbtes Plättchen liefert. Die Röntgensichtbarkeit beträgt etwa 70 % A1 bei etwas weniger als 5 % Ba.
6. Herstellung von röntgensichtbarem, Barium-haltigen Polymerisat II

| | |
|---|---|
| 2,49 g | Bariumhydroxid, wasserfrei |
| 6,03 g | Methacrylsäure |
| 0,54 g | Phenylessigsäure |
| 6,67 g | Urethandimethacrylat |
| 0,36 g | TPO |

Die Mischung ergibt eine klare, leicht gelbliche Lösung, die nach der Polymerisationsreaktion ein glasklares, leicht gelblich gefärbtes Plättchen liefert, durch das problemlos gelesen werden kann. Die Röntgensichtbarkeit beträgt etwa 100 % A1 bei etwas weniger als 10 % Ba.
7. Herstellung Holmium-haltigem Polymerisat geeignet für Anwendungen in der Optik und zur Oberflächenbeschichtung

| | |
|---|---|
| 1,19 g | Holmiumcarbonat |
| 4,04 g | 3-Phenylpropionsäure |
| 2,17 g | Methacrylsäure |
| 0,66 g | *iso*-Bornylmethacrylat |
| 5,88 g | Urethandimethacrylat |
| 0,36 g | TPO-L |

Die Mischung ergibt eine klare, gelbliche Lösung, die nach der Polymerisationsreaktion ein klares, gelblich gefärbtes Plättchen liefert. Die Röntgensichtbarkeit beträgt etwa 67 % A1 bei etwas weniger als 5 % Ho.
Bei der Belichtung mit Kunstlicht sind Monomerengemisch und Plättchen deutlich rosa gefärbt, während sie bei Tageslicht lediglich hellgelb (Fig. 1, Probekörper G) erscheinen.

8. Herstellung eines Zirkonium-haltigem Polymerisats (nicht erfindungsgemäß)

| | |
|---|---|
| 2,27 g | Zirkonium(IV)-methacrylat |
| 3,29 g | Tetrahydrofurfurylmethacrylat |
| 1,06 g | Methacrylsäure |
| 2,05 g | Triethylenglycoldimethacrylat |
| 7,41 g | Phenylglyceroldimethacrylat |
| 0,36 g | TPO-L |

Die Mischung ergibt eine klare, gelbliche Lösung, die nach der Polymerisationsreaktion ein klares, gelblich gefärbtes Plättchen liefert.
9. Herstellung eines Strontium-haltigem Polymerisats (nicht erfindungsgemäß)

| | |
|---|---|
| 0,28 g | Strontiumhydroxid |
| 4,39 g | Tetrahydrofurfurylmethacrylat |
| 2,04 g | Methacrylsäure |
| 2,71 g | Mono-2-methacryloyloxyethylsuccinat |
| 5,95 g | Bisphenol-A-glycidylmethacrylat |
| 0,32 g | TPO-L |

Die Mischung ergibt eine klare, fast farblose Lösung, die nach der Polymerisationsreaktion ein klares Plättchen liefert.

Somit betrifft die vorliegende Erfindung ein härtbares röntgensichtbares Material zur Herstellung eines Werkstoffes, herstellbar durch Mischen von Ausgangsmaterialien, wobei als zu mischende Ausgangsmaterialien eingesetzt werden:
- ein oder mehrere polymerisierbare(s) Monomer(e), eine oder mehrere Strontium-, Zirkonium-, Blei-, Barium-, Bismut- oder Seltenerdverbindung(en), die in dem Monomeren oder in dem Monomerengemisch löslich ist/sind,
- ein oder mehrere Härtungsinitiator(en) und
- ein oder mehrere Hilfsstoffe, dadurch gekennzeichnet, dass der Hilfsstoff eine aromatische Carbonsäure oder eine Aralkylcarbonsäure ist, womit die Aralkylcarbonsäure eine substituierte oder unsubstituierte, gesättigte oder ungesättigte Carbonsäure ist, womit vorzugsweise die Carbonsäure Phenylessigsäure oder 3-Phenylpropionsäure oder trans-Zimtsäure ist.

Bevorzugt betrifft die vorliegende Erfindung ein härtbares Material, worin wenigstens eines der polymerisierbaren Monomere aus der Gruppe der radikalisch härtbaren Monomere ausgewählt ist.

Weiterhin bevorzugt betrifft die vorliegende Erfindung ein härtbares Material, worin wenigstens eines der polymerisierbaren Monomere aus der Gruppe der Acrylsäure, Acrylate, Methacrylsäure oder Methacrylate bzw. aus deren Derivaten ausgewählt ist.

Weiterhin bevorzugt betrifft die vorliegende Erfindung ein härtbares Material, worin das Acrylsäurederivat und/oder das Methacrylsäurederivat ausgewählt ist aus der Gruppe umfassend Acrylsäureester, Methacrylsäureester, Acrylamid oder Methacrylamid.

Weiterhin bevorzugt betrifft die vorliegende Erfindung ein härtbares Material, worin das Acrylsäure- und/oder Methacrylsäurederivat ausgewählt ist aus der Gruppe umfassend Methacrylsäure, Butyldiglycolmethacrylat, Urethandimethacrylat, iso-Bomylmethacrylat, Tetrahydrofurfuryl-methacrylat, 1,4-Butandioldimethacrylat, 2-[[(Butylamino)carbonyl]oxy]-ethylacrylat, Bisphenol-A-dimethacrylat und/oder Methylmethacrylat.

Weiterhin bevorzugt betrifft die vorliegende Erfindung ein härtbares Material, worin die Blei-, Barium-, Wismut- oder Seltenerdverbindung eine polymerisierbare Strontium-, Zirkonium-, Blei-, Barium-, Wismut- oder Seltenerdverbindung und/oder ein anorganisches oder organisches Seltenerdsalz oder eine Komplexverbindung ist.

Weiterhin bevorzugt betrifft die vorliegende Erfindung ein härtbares Material, worin die Blei-, Barium-, Wismut- und/oder Seltenerdverbindung in einer Konzentration enthalten ist, die eine Röntgensichtbarkeit ermöglicht.

Weiterhin bevorzugt betrifft die vorliegende Erfindung ein härtbares Material, worin der Härtungsinitiator ein UV-Härtungsinitiator ist.

Weiterhin bevorzugt betrifft die vorliegende Erfindung ein härtbares Material, worin der Härtungsinitiator aus einem Redox-2-Komponenten-System besteht und als Hilfsstoff eine pulverförmige Komponente enthält, die nach dem Vermischen mit der flüssigen Komponente ein selbsthärtendes Material ergibt.

Weiterhin bevorzugt betrifft die vorliegende Erfindung ein härtbares Material, worin der UV-Initiator aus der Gruppe der Phosphinoxide, bevorzugt Diphenyl(2,4,6-trimethylbenzoyl)phosphinoxid (TPO) und/oder 2,4,6-Trimethylbenzoylphenyl-phosphinat (TPO-L) und/oder Bis(2,4,6-trimethylbenzoyl)-phenylphosphinoxid (BAPO) und/oder Campherchinon und/oder eine Verbindung aus der Gruppe der Thioxanthone ist.

Der Hilfsstoff im härtbaren Material der vorliegenden Erfindung ist eine aromatische oder eine Aralkylcarbonsäure.

Weiterhin bevorzugt betrifft die vorliegende Erfindung ein härtbares Material, worin das Carbonsäurederivat Phenylessigsäure und/oder 3-Phenylpropionsäure und/oder trans-Zimtsäure ist.

Weiterhin bevorzugt betrifft die vorliegende Erfindung ein härtbares Material, worin die polymerisierbare Partialstruktur durch wenigstens eine radikalisch polymerisierbare Gruppe dargestellt wird.

Weiterhin bevorzugt betrifft die vorliegende Erfindung ein härtbares Material, worin der Komplexbildner ausgewählt ist aus der Gruppe umfassend 2-Methacryloyloxyethylacetoacetat (AAEMA), Bis(2-methacryloyloxyethyl)-pyromellitat, Methacryloyloxyethylphthalat, Methacryloyloxyethylmaleat, Methacryloyloxyethylsuccinat und/oder deren Derivate.

Weiterhin bevorzugt betrifft die vorliegende Erfindung ein härtbares Material, worin der Komplexbildner, bevorzugt jedoch das härtbare Gemisch insgesamt, bei Raumtemperatur einen Dampfdruck von kleiner 1 mbar bei 20 °C, besonders bevorzugt von kleiner 0,3 mbar bei 20 °C und ganz besonders bevorzugt einen Dampfdruck von kleiner 0,1 mbar bei 20 °C besitzt.

Weiterhin bevorzugt betrifft die vorliegende Erfindung ein härtbares Material, worin das härtbare Material nach der Polymerisation einen für elektromagnetische Wellen im Bereich des sichtbaren Lichtes transparenten, ggf. auf Grund der enthaltenen Metallionen gefärbten, Werkstoff ergibt.

Weiterhin bevorzugt betrifft die vorliegende Erfindung ein härtbares Material zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird, vorzugsweise zur spezifischen Anwendung
- in einem therapeutischen Verfahren zum temporären oder permanenten Füllen einer dentalen Kavität sowie in einem therapeutischen Verfahren als
- Zahnfüllungsmaterial,
- Dentalzement,
- dentales Unterfüllungsmaterial,
- als fließfähiges Kompositmaterial (Flow-Material),
- als Kronenmaterial,
- als Inlay und/oder Onlay,
- als Bohrschablone
- und/oder als Stumpfaufbaumaterial
   und/oder in einem diagnostischen Verfahren als
- Bohrschablone
- Röntgenkontrastierungsmittel.

Daneben betrifft die vorliegende Erfindung ein Verfahren zur Herstellung eines härtbaren Materials umfassend die folgenden Schritte:
(i) Herstellen oder Bereitstellen der Ausgangsmaterialien wie in einem der Ansprüche 1 bis 21 definiert,
   oder
   Herstellen oder Bereitstellen von Vorprodukten aus den Ausgangsmaterialien wie vorstehend definiert
(ii) Mischen der gemäß Schritt (i) hergestellten bzw. bereitgestellten Ausgangsmaterialien bzw. der gemäß (i) hergestellten bzw. bereitgestellten Vorprodukte, so dass jeweils das härtbare Material resultiert.

Des Weiteren betrifft die vorliegende Erfindung bevorzugt die Verwendung eines härtbaren Materials - wie vorstehend definiert - im 3D-Druck.

Des Weiteren betrifft die vorliegende Erfindung besonders bevorzugt die Verwendung eines härtbaren Materials - wie vorstehend definiert - als Baumaterial in einem generativen, ein digitales Datenmodell verwendenden Fertigungsverfahren.

Des Weiteren betriff die vorliegende Erfindung ganz besonders bevorzugt die Verwendung des härtbaren Materials - wie vorstehend definiert - zur Herstellung eines dentalen Erzeugnisses, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen sowie Bohrschablonen.

Daneben betrifft die vorliegende Erfindung die Verwendung eines härtbaren Materials zur Herstellung eines Erzeugnisses für die Verwendung im Bereich der Optik, vorzugsweise zur Herstellung von Linsen und/oder Filtern.

Des Weiteren betrifft die vorliegende Erfindung ein Verfahren zur Herstellung eines dentalen Erzeugnisses mittels eines generativen, ein digitales Datenmodell verwendenden Fertigungsverfahrens umfassend die Schritte:
(i) Herstellen oder Bereitstellen eines härtbaren Dentalmaterials wie zuvor definiert, vorzugsweise Herstellen nach dem oben erwähnten Verfahren
   und
(ii) Verarbeiten des hergestellten bzw. bereitgestellten härtbaren Dentalmaterials in dem generativen, ein digitales Datenmodell verwendenden Fertigungsverfahren, so dass das dentale Erzeugnis oder eine Vorstufe des dentalen Erzeugnisses resultiert,
wobei das dentale Erzeugnis vorzugsweise ausgewählt ist aus der Gruppe bestehend aus künstlichen Zähnen, Inlays, Onlays, Kronen, Brücken, Fräsrohlingen, Implantaten, Bohrschablonen und Zahnfertigteilen.

Des Weiteren betrifft die vorliegende Erfindung ein gehärtetes Material bzw. einen Werkstoff, erhältlich durch Polymerisation polymerisierbarer Monomere in einem härtbaren Material wie vorstehend definiert.

Des Weiteren betrifft die vorliegende Erfindung ein Kit, umfassend
- eine oder mehr als eine Spritze und
   (i) ein, zwei oder mehr als zwei härtbare Materialien wie vorstehend definiert und/oder
   (ii) eine, zwei oder mehr als zwei Basispasten und eine, zwei oder mehr Katalysatorpasten, wobei ein härtbares Material - wie vorstehend definiert - erhältlich ist durch Mischen einer Basispaste und der dazugehörigen Katalysatorpaste.

## Patentansprüche

1. Härtbares röntgensichtbares Material zur Herstellung eines Werkstoffes, herstellbar durch Mischen von Ausgangsmaterialien, wobei als zu mischende Ausgangsmaterialien eingesetzt werden:
- ein oder mehrere polymerisierbare(s) Monomer(e),
- eine oder mehrere Strontium-, Zirkonium-, Blei-, Barium-, Wismut- oder Seltenerdverbindung(en), die in dem Monomeren oder in dem Monomerengemisch löslich ist / sind,
- ein oder mehrere Härtungsinitiator(en) und
- ein oder mehrere Hilfsstoffe,
**dadurch gekennzeichnet, dass** der Hilfsstoff eine aromatische Carbonsäure oder eine Aralkylcarbonsäure ist, womit die Aralkylcarbonsäure eine substituierte oder unsubstituierte, gesättigte oder ungesättigte Carbonsäure ist, womit vorzugsweise die Carbonsäure Phenylessigsäure oder 3-Phenylpropionsäure oder trans-Zimtsäure ist.

2. Härtbares Material nach Anspruch 1, **dadurch gekennzeichnet, dass** wenigstens eines der polymerisierbaren Monomere aus der Gruppe der Acrylsäure, Acrylate, Methacrylsäure oder Methacrylate bzw. aus deren Derivaten ausgewählt ist, womit vorzugsweise das Acrylsäurederivat oder das Methacrylsäurederivat ausgewählt ist aus der Gruppe umfassend Acrylsäureester, Methacrylsäureester, Acrylamid oder Methacrylamid, womit vorzugsweise das Acrylsäure- oder Methacrylsäurederivat ausgewählt ist aus der Gruppe umfassend Methacrylsäure, Butyldiglycolmethacrylat, Urethandimethacrylat, iso-Bomylmethacrylat, Tetrahydrofurfuryl-methacrylat, 1,4-Butandioldimethacrylat, 2-[[(Butylamino)carbonyl]oxy]ethylacrylat, Bisphenol-A-dimethacrylat und Methylmethacrylat.

3. Härtbares Material nach Anspruch 1 oder 2, **dadurch gekennzeichnet, dass** die Strontium-, Zirkonium-, Blei-, Barium-, Wismut- oder Seltenerdverbindung eine polymerisierbare Strontium-, Zirkonium-, Blei-, Barium-, Wismut- oder Seltenerdverbindung oder ein anorganisches oder organisches Seltenerdsalz oder eine Komplexverbindung ist, womit vorzugsweise die Strontium-, Zirkonium-, Blei-, Barium-, Wismut- oder Seltenerdverbindung in einer Konzentration enthalten ist, die eine Röntgensichtbarkeit ermöglicht, und vorzugsweise der Härtungsinitiator ein UV-Härtungsinitiator ist, oder vorzugsweise der Härtungsinitiator aus einem Redox-2-Komponenten-System besteht und als Hilfsstoff eine pulverförmige Komponente enthält, die nach dem Vermischen mit der flüssigen Komponente ein selbsthärtendes Material ergibt.

4. Härtbares Material nach einem der Ansprüche 1 bis 3, **dadurch gekennzeichnet, dass** der UV-Initiator aus der Gruppe umfassend Phosphinoxide, Thioxanthone sowie Campherchinon ausgewählt ist., vorzugsweise der UV-Initiator ausgewählt ist aus der Gruppe umfassend Diphenyl(2,4,6-trimethylbenzoyl)phosphinoxid (TPO), 2,4,6-Trimethylbenzoylphenylphosphinat (TPO-L) und Bis(2,4,6-trimethylbenzoyl)phenyl-phosphinoxid (BAPO).

5. Härtbares Material nach einem der Ansprüche 1 bis 4, **dadurch gekennzeichnet, dass** das Monomerengemisch bei Raumtemperatur einen Dampfdruck von kleiner 1 mbar / 20 °C, aufweist, und vorzugsweise das härtbare Gemisch bei Raumtemperatur einen Dampfdruck von kleiner von kleiner 0,3 mbar / 20 °C aufweist, und vorzugsweise das härtbare Gemisch bei Raumtemperatur einen Dampfdruck von kleiner 0,1 mbar / 20 °C aufweist.

6. Härtbares Material nach Anspruch 1 bis 5, **dadurch gekennzeichnet, dass** das härtbare Material nach der Polymerisation einen für elektromagnetische Wellen im Bereich des sichtbaren Lichtes transparenten, farblosen oder gefärbten, Werkstoff ergibt.

7. Härtbares Material nach einem der Ansprüche 1 bis 6 zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird, vorzugsweise zur spezifischen Anwendung
- in einem therapeutischen Verfahren zum temporären oder permanenten Füllen einer dentalen Kavität sowie in einem therapeutischen Verfahren als
- Zahnfüllungsmaterial,
- Dentalzement,
- dentales Unterfüllungsmaterial,
- als fließfähiges Kompositmaterial (Flow-Material),
- als Kronenmaterial,
- als Inlay oder Onlay,
- als Bohrschablone
- oder als Stumpfaufbaumaterial
und/oder in einem diagnostischen Verfahren als
- Bohrschablone
- Röntgenkontrastierungsmittel.

8. Verfahren zur Herstellung eines härtbaren Dentalmaterials nach einem der Ansprüche 1 bis 6 umfassend die folgenden Schritte:
(i) Herstellen oder Bereitstellen der Ausgangsmaterialien wie in einem der Ansprüche 1 bis 6 definiert,
oder
Herstellen oder Bereitstellen von Vorprodukten aus den Ausgangsmaterialien wie in einem der Ansprüche 1 bis 7 definiert
(ii) Mischen der gemäß Schritt (i) hergestellten bzw. bereitgestellten Ausgangsmaterialien bzw. der gemäß (i) hergestellten bzw. bereitgestellten Vorprodukte, so dass jeweils das härtbare Dentalmaterial resultiert.

9. Verwendung eines härtbaren Materials nach einem der Ansprüche 1 bis 6 als Baumaterial in einem generativen, ein digitales Datenmodell verwendenden Fertigungsverfahren, und vorzugsweise im 3D-Drcuk oder vorzugsweise zur Herstellung eines dentalen Erzeugnisses, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen, vorzugsweise zur Herstellung eines Erzeugnisses für die Verwendung im Bereich der Optik, und vorzugsweise zur Herstellung von Linsen und/oder Filtern.

10. Verfahren zur Herstellung eines dentalen Erzeugnisses mittels eines generativen, ein digitales Datenmodell verwendenden Fertigungsverfahrens umfassend die Schritte:
(i) Herstellen oder Bereitstellen eines härtbaren Materials nach einem der Ansprüche 1 bis 6,
(ii) Verarbeiten des hergestellten bzw. bereitgestellten härtbaren Materials in dem generativen, ein digitales Datenmodell verwendenden Fertigungsverfahren, so dass das dentale Erzeugnis oder eine Vorstufe des dentalen Erzeugnisses resultiert, und vorzugsweise das dentale Erzeugnis ausgewählt ist aus der Gruppe bestehend aus künstlichen Zähnen, Inlays, Onlays, Kronen, Brücken, Fräsrohlingen, Implantaten, Bohrschablonen und Zahnfertigteilen.

11. Gehärtetes Material, erhältlich durch Polymerisation polymerisierbarer Monomere in einem härtbaren Material nach einem der Ansprüche 1 bis 6.

12. Kit, umfassend
- eine oder mehr als eine Spritze und
(i) ein, zwei oder mehr als zwei härtbare Materialien nach einem der Ansprüche 1 bis 6 und/oder
(ii) eine, zwei oder mehr als zwei Basispasten und eine, zwei oder mehr Katalysatorpasten, wobei ein härtbares Material nach einem der Ansprüche 1 bis 6 erhältlich ist durch Mischen einer Basispaste und der dazugehörigen Katalysatorpaste.

## Claims

1. Curable X-ray visible material for producing a material, which can be produced by mixing starting materials, wherein the following starting materials are used for mixing:
- one or more polymerisable monomer(s),
- one or more strontium, zirconium, lead, barium, bismuth or rare earth compounds which are soluble in the monomer or in the monomer mixture,
- one or more curing initiators, and
- one or more auxiliary substances,
**characterised in that** the auxiliary material is an aromatic carboxylic acid or an aralkyl carboxylic acid, wherein the aralkyl carboxylic acid is a substituted or unsubstituted, saturated or unsaturated carboxylic acid, wherein the carboxylic acid is preferably phenylacetic acid or 3-phenylpropionic acid or trans-cinnamic acid.

2. Curable material according to claim 1, **characterised in that** at least one of the polymerisable monomers is selected from the group consisting of acrylic acid, acrylates, methacrylic acid or methacrylates or theirderivatives, wherein preferably the acrylic acid derivative or the methacrylic acid derivative is selected from the group comprising acrylic acid esters, methacrylic acid esters, acrylamide or methacrylamide, whereby the acrylic acid or methacrylic acid derivative is preferably selected from the group comprising methacrylic acid, butyldiglycol methacrylate, urethane dimethacrylate, iso-bornyl methacrylate, tetrahydrofurfuryl methacrylate, 1,4-butanediol dimethacrylate, 2-[[(butylamino)carbonyl] oxy] ethyl acrylate, bisphenol-A-dimethacrylate and methyl methacrylate.

3. Curable material according to claim 1 or 2, **characterised in that** the strontium, zirconium, lead, barium, bismuth or rare earth compound is a polymerisable strontium, zirconium, lead, barium, bismuth or rare earth compound or an inorganic or organic rare earth salt or a complex compound, whereby the strontium, zirconium, lead, barium, bismuthor rare earth compound is preferably contained in a concentration that enables X-ray visibility, and preferably the curing initiator is a UV curing initiator, or preferably the curing initiator consists of a redox-2-component system and contains a powdered component as an auxiliary material, which, after mixing with the liquid component, produces a self-curing material.

4. Curable material according to any one of claims 1 to 3, **characterised in that** the UV initiator is selected from the group comprising phosphine oxides, thioxanthones and camphorquinone, preferably the UV initiator is selected from the group comprising diphenyl(2,4,6-trimethylbenzoyl)phosphine oxide (TPO), 2,4,6-trimethylbenzoyl/phenylphosphinate (TPO-L) and bis(2,4,6-trimethylbenzoyl)phenylphosphine oxide (BAPO).

5. Curable material according to any one of claims 1 to 4, **characterised in that** the monomer mixture has a vapour pressure at room temperature of less than 1 mbar/20 °C, and preferably the curable mixture has a vapour pressure at room temperature of less than 0.3 mbar / 20 °C, and preferably the curable mixture has a vapour pressure at room temperature of less than 0.1 mbar / 20 °C.

6. Curable material according to claims 1 to 5, **characterised in that** after polymerisation, the curable material yields a material that is transparent to electromagnetic waves in the visible light range, colourless or coloured.

7. Curable material according to one of claims 1 to 6 for use in a method for the surgical or therapeutic treatment of the human or animal body and/or for use in a diagnostic method performed on the human or animal body, preferably for specific use.
- in a therapeutic procedure for temporarily or permanently filling a dental cavity, as well as in a therapeutic procedure as
- dental filling material,
- dental cement,
- dental underfill material,
- as a flowable composite material (flow material),
- as a crown material,
- as an inlay or onlay,
- as a drilling template
- or as a core build-up material
and/or in a diagnostic procedure as
- drilling template
- X-ray contrast medium.

8. Method for producing a curable dental material according to one of claims 1 to 6, comprising the following steps:
(i) producing or providing the starting materials as defined in one of claims 1 to 6,
or
producing or providing preliminary products from the starting materials as defined in one of claims 1 to 7
(ii) mixing the starting materials produced or provided in accordance with step (i) or the preliminary products produced or provided in accordance with (i) so that the curable dental material results in each case.

9. Use of a curable material according to one of claims 1 to 6 as a building material in a generative manufacturing process using a digital data model, and preferably in 3D printing or preferably for the manufacture of a dental product, preferably for the manufacture of a dental product selected from the group consisting of artificial teeth, inlays, onlays, crowns, bridges, milling blanks, implants and prefabricated dental parts, preferably for the manufacture of a product for use in the field of optics, and preferably for the manufacture of lenses and/or filters.

10. Method for manufacturing a dental product by means of a generative manufacturing process using a digital data model, comprising the steps:
(i) producing or providing a curable material according to one of claims 1 to 6,
(ii) processing the manufactured or provided curable material in the generative manufacturing process using a digital data model so that the dental product or a precursor of the dental product results, and preferably the dental product is selected from the group consisting of artificial teeth, inlays, onlays, crowns, bridges, milling blanks, implants, drilling templates and prefabricated dental parts.

11. Cured material obtainable by polymerisation of polymerisable monomers in a curable material according to any one of claims 1 to 6.

12. Kit comprising
- one or more syringes and
(i) one, two or more than two curable materials according to any one of claims 1 to 6 and/or
(ii) one, two or more than two base pastes and one, two or more catalyst pastes, wherein a curable material according to any one of claims 1 to 6 can be obtained by mixing a base paste and the corresponding catalyst paste.

## Revendications

1. Matériau durcissable visible aux rayons X pour la fabrication d'un matériau, pouvant être fabriqué par mélange de matières premières, les matières premières à mélanger utilisées étant :
- un ou plusieurs monomères polymérisables,
- un ou plusieurs composés de strontium, de zirconium, de plomb, de baryum, de bismuth ou de terres rares, qui sont solubles dans le monomère ou dans le mélange de monomères,
- un ou plusieurs initiateurs de durcissement et
- un ou plusieurs adjuvants,
**caractérisé en ce que** l'adjuvant est un acide carboxylique aromatique ou un acide aralkylcarboxylique, l'acide aralkylcarboxylique étant un acide carboxylique substitué ou non substitué, saturé ou insaturé, l'acide carboxylique étant de préférence l'acide phénylacétique ou l'acide 3-phénylpropionique ou l'acide trans-cinnamique.

2. Matériau durcissable selon la revendication 1, **caractérisé en ce qu'**au moins l'un des monomères polymérisables est choisi dans le groupe constitué par l'acide acrylique, les acrylates, l'acide méthacrylique ou les méthacrylates ou leurs dérivés, le dérivé d'acide acrylique ou le dérivé d'acide méthacrylique étant de préférence choisi dans le groupe comprenant les esters d'acide acrylique, les les esters d'acide méthacrylique, l'acrylamide ou le méthacrylamide, le dérivé d'acide acrylique ou d'acide méthacrylique étant de préférence choisi parmi le groupe comprenant l'acide méthacrylique, le méthacrylate de butyldiglycol, méthacrylate d'uréthane, méthacrylate d'iso-bornyle, méthacrylate de tétrahydrofurfuryle, méthacrylate de 1,4-butanediol, méthacrylate de 2-[[[(butylamino)carbonyl] oxy] éthyle, méthacrylate de bisphénol A et méthacrylate de méthyle.

3. Matériau durcissable selon la revendication 1 ou 2, **caractérisé en ce que** le composé de strontium, de zirconium, de plomb, de baryum, de bismuth ou de terre rare est un composé polymérisable de strontium, zirconium, de plomb, de baryum, de bismuth ou de terre rare polymérisable ou un sel de terre rare inorganique ou organique ou un composé complexe, de sorte que le composé de strontium, de zirconium, de plomb, de baryum, de bismuthou de terre rare est contenue dans une concentration qui permet la visibilité aux rayons X, et de préférence l'initiateur de durcissement est un initiateur de durcissement aux UV, ou de préférence l'initiateur de durcissement est constitué d'un système redox à deux composants et contient comme adjuvant un composant pulvérulent qui, après mélange avec le composant liquide, donne un matériau autodurcissant.

4. Matériau durcissable selon l'une des revendications 1 à 3, **caractérisé en ce que** l'initiateur UV est choisi dans le groupe comprenant les oxydes de phosphine, les thioxanthones et la camphoquinone, de préférence l'initiateur UV est choisi dans le groupe comprenant l'oxyde de diphényl(2,4,6-triméthylbenzoyl)phosphine (TPO), du 2,4,6-triméthylbenzoyl/phénylphosphinate (TPO-L) et de l'oxyde de bis(2,4,6-triméthylbenzoyl)phénylphosphine (BAPO).

5. Matériau durcissable selon l'une des revendications 1 à 4, **caractérisé en ce que** le mélange de monomères présente à température ambiante une pression de vapeur inférieure à 1 mbar / 20 °C, et de préférence que le mélange durcissable présente à température ambiante une pression de vapeur inférieure à 0,3 mbar / 20 °C, et de préférence que le mélange durcissable présente à température ambiante une pression de vapeur inférieure à 0,1 mbar / 20 °C.

6. Matériau durcissable selon les revendications 1 à 5, **caractérisé en ce que** le matériau durcissable donne après polymérisation un matériau transparent, incolore ou coloré, pour les ondes électromagnétiques dans le domaine de la lumière visible.

7. Matériau durcissable selon l'une des revendications 1 à 6, destiné à être utilisé dans un procédé de traitement chirurgical ou thérapeutique du corps humain ou animal et/ou dans un procédé de diagnostic effectué sur le corps humain ou animal, de préférence pour une application spécifique.
- dans un procédé thérapeutique pour le comblement temporaire ou permanent d'une cavité dentaire ainsi que dans un procédé thérapeutique en tant que
- matériau d'obturation dentaire,
- ciment dentaire,
- matériau de remplissage dentaire,
- comme matériau composite fluide (matériau fluide),
- comme matériau pour couronnes,
- comme inlay ou onlay,
- comme gabarit de perçage
- ou comme matériau de reconstitution de moignon
et/ou dans une procédure diagnostique comme
- gabarit de perçage
- agent de contraste radiographique.

8. Procédé de fabrication d'un matériau dentaire durcissable selon l'une des revendications 1 à 6, comprenant les étapes suivantes :
(i) la fabrication ou la fourniture des matériaux de départ tels que définis dans l'une des revendications 1 à 6,
ou
la fabrication ou la mise à disposition de produits intermédiaires à partir des matières premières telles que définies dans l'une des revendications 1 à 7
(ii) Mélange des matières premières fabriquées ou fournies conformément à l'étape (i) ou des produits préliminaires fabriqués ou fournis conformément à (i), de manière à obtenir le matériau dentaire durcissable.

9. Utilisation d'un matériau durcissable selon l'une des revendications 1 à 6 comme matériau de construction dans un procédé de fabrication génératif utilisant un modèle de données numériques, et de préférence dans l'impression 3D ou de préférence pour la fabrication d'un produit dentaire, de préférence pour la fabrication d'un produit dentaire choisi dans le groupe constitué par les dents artificielles, les inlays, onlays, couronnes, bridges, ébauches fraisées, implants et pièces dentaires préfabriquées, de préférence pour la fabrication d'un produit destiné à être utilisé dans le domaine de l'optique, et de préférence pour la fabrication de lentilles et/ou de filtres.

10. Procédé de fabrication d'un produit dentaire au moyen d'un procédé de fabrication génératif utilisant un modèle de données numériques, comprenant les étapes suivantes:
(i) la fabrication ou la fourniture d'un matériau durcissable selon l'une des revendications 1 à 6,
(ii) traitement du matériau durcissable fabriqué ou fourni dans le procédé de fabrication génératif utilisant un modèle de données numériques, de manière à obtenir le produit dentaire ou un précurseur du produit dentaire, et de préférence le produit dentaire est choisi dans le groupe constitué par les dents artificielles, les inlays, onlays, couronnes, bridges, ébauches fraisées, implants, gabarits de perçage et pièces dentaires préfabriquées.

11. Matériau durci, pouvant être obtenu par polymérisation de monomères polymérisables dans un matériau durcissable selon l'une des revendications 1 à 6.

12. Kit comprenant
- une ou plusieurs seringues et
(i) un, deux ou plus de deux matériaux durcissables selon l'une des revendications 1 à 6 et/ou
(ii) une, deux ou plusieurs pâtes de base et une, deux ou plusieurs pâtes catalytiques, un matériau durcissable selon l'une des revendications 1 à 6 pouvant être obtenu en mélangeant une pâte de base et la pâte catalytique correspondante.
